# FASCICULE DE BREVET EUROPEEN

(11) **EP 1 663 102 B1**
(45) Date de publication et mention de la délivrance du brevet: **14.04.2010**
(21) Numéro de dépôt: 04786307.1
(22) Date de dépôt: 12.08.2004
(51) Int. Cl.: A61J 7/00, A61J 1/00, A61M 5/178

(54) **DISPOSITIF D'ADMINISTRATION ORALE D'UN MEDICAMENT**
VORRICHTUNG ZUR ORALEN VERABREICHUNG EINES MEDIKAMENTS
DEVICE FOR ORAL ADMINISTRATION OF A MEDICAMENT

(30) Priorité: 21.08.2003 FR 0310079
(43) Date de publication de la demande: 07.06.2006
(73) Titulaire: Becton Dickinson France, 38800 Le Pont-de-Claix (FR)
(72) Inventeur: BARRELLE, Laurent, F-38250 Saint Nizier du Moucherotte (FR); PEROT, Frédéric, 38760 Saint Paul de Varces (FR); FELIX-FAURE, Catherine, F-38000 Grenoble (FR)
(74) Mandataire: Brédeville, Odile Marie
(86) Numéro de dépôt international: PCT/FR2004/002137
(87) Numéro de publication internationale: WO 2005/020875

(56) Documents cités:
- EP-A- 0 764 450
- FR-A- 2 653 667
- FR-A- 2 821 561
- US-A- 4 393 864
- US-A- 4 516 967
- US-A- 5 244 122
- US-A- 5 417 660
- US-A- 5 876 379
- US-A- 5 893 397
- US-A- 5 925 032
- US-A1- 2002 045 864

## Description

La présente invention concerne un ensemble pour l'administration orale d'un produit, par exemple médicamenteux, à reconstituer à partir d'un principe actif sous forme sèche ou liquide, comprenant un flacon comprenant ledit actif, un système de transfert et un dispositif comprenant un support et une seringue.

Il est connu d'utiliser une seringue pour l'administration orale de médicaments. Cette méthode s'avère particulièrement pratique et avantageuse chez les enfants et les personnes âgées. De façon classique, une telle seringue comprend un corps tubulaire, un embout et un piston. Le médicament, contenu dans un flacon à part, est aspiré dans le corps de la seringue par retrait du piston vers l'extrémité proximale de la seringue puis ensuite injecté dans la bouche du patient en poussant le piston vers l'extrémité distale de la seringue.

Toutefois, dans certains cas, il est nécessaire que la seringue soit pré-remplie. Par exemple, la seringue peut contenir un diluant d'un principe actif sous forme sèche, comme une poudre ou un lyophilisat, ou sous forme liquide. Dans ce cas, la seringue doit d'abord être connectée, de préférence par son embout, au flacon contenant le principe actif en poudre par exemple, au moyen d'un système de transfert, puis le produit médicamenteux est reconstitué par mélange dudit principe actif avec le diluant déjà présent dans la seringue. Le produit médicamenteux peut ensuite être introduit dans la bouche du patient.

La seringue peut également être directement pré-remplie par un médicament sous forme liquide.

Dans le cas de certains produits, il est particulièrement important que le contenu de la seringue, que ce soit le médicament, le diluant ou le produit médicamenteux reconstitué, ne puisse être injecté au patient par voie parentérale, par exemple de façon sous cutanée ou intraveineuse au moyen d'un organe d'injection parentérale, comme une aiguille, un cathether ou une ligne de perfusion. En effet, une telle injection pourrait être mortelle pour le patient.

Il est également intéressant d'un point de vue pratique de pouvoir utiliser des seringues pré-remplies standard pour une administration orale de produit médicamenteux.

Par ailleurs, lorsque la seringue utilisée est en verre, il est important, en particulier lorsque le patient est un enfant, que cette seringue n'entre pas en contact avec la bouche de l'enfant par exemple, ou avec tout autre partie du corps, qu'elle pourrait blesser en cas de brisure. Dans ce cas, il est également important que la seringue soit protégée des chocs.

Ainsi, il existe le besoin d'un dispositif sécurisé d'administration orale de produit médicamenteux utilisant des seringues pré-remplies, en particulier des seringues pré-remplies standard, et par exemple en verre, autorisant d'une part une administration orale du produit et interdisant d'autre part l'administration parentérale de ce même produit. L'ensemble comprenant un tel dispositif, le flacon comprenant le principe actif sous forme sèche ou liquide, et le système de transfert, doit ainsi interdire l'injection parentérale de tout produit tout en permettant la connexion du système de transfert à la seringue.

Le document WO94/13347 décrit un dispositif comprenant en combinaison une seringue pré-remplie et un support pouvant être fixé sur la seringue en vue d'empêcher la contamination de cette dernière lors de son utilisation. Ce dispositif n'interdit pas la connexion d'un organe d'injection parentérale à l'embout de la seringue et n'interdit donc pas non plus l'administration parentérale du produit contenu dans la seringue.

Le document US 4, 516, 967 décrit un ensemble pour l'administration parentérale d'un produit médicamenteux à reconstituer à partir d'un principe actif sous forme sèche et d'un diluant comprenant une seringue pré-remplie munie d'une aiguille, un flacon comprenant le principe actif sous forme sèche et un système de transfert, sous la forme d'un tube en plastique, reliant la seringue et le flacon. Dans US 4, 516, 967, une fois le médicament reconstitué, et le flacon et le système de transfert retirés, la seringue munie de son aiguille est prête à être utilisée en injection parentérale.

De façon classique, un organe d'injection parentérale est fourni muni d'une embase de connexion. Ainsi, classiquement, une aiguille d'injection amovible est constituée d'une aiguille tubulaire généralement métallique fixée sur une embase, par exemple en plastique en forme de cône creux, destinée à venir en contact avec l'embout de la seringue, qui est lui-même conique, tel que l'assemblage dit « Luer » connu en soi. Ce cône creux est muni généralement de deux ailettes à sa base de façon à s'adapter à une partie taraudée de l'organe d'injection parentérale de façon à assurer le maintien en contact sans fuite de l'embase sur l'embout.

Pour interdire l'utilisation par voie parentérale d'une seringue dans l'application orale désirée, il est nécessaire d'empêcher toute connexion potentielle d'un organe d'injection parentérale, en particulier du type « Luer», au niveau de l'embout de la seringue. En particulier, une connexion de l'organe par mise en contact et/ou vissage de son embase doit être impossible. Toutefois, la connexion du système de transfert à la seringue doit rester possible.

La présente invention vise à remédier à ces problèmes en fournissant un ensemble sécurisé d'administration orale de produit médicamenteux permettant l'utilisation de seringues pré-remplies standard dans la reconstitution du médicament à partir d'un principe actif sous forme sèche ou liquide par connexion du système de transfert à ta seringue, puis l'administration orale du produit tout en interdisant l'administration parentérale de ce même produit.

Un objet de la présente invention, tel que défini dans la revendication 1, est un ensemble pour l'administration orale d'un produit médicamenteux à reconstituer à partir d'un principe actif sous forme sèche ou liquide et d'un diluant, **caractérisé en ce qu**'il comprend :
- i) un dispositif comprenant :
   - une seringue pré-remplie avec ledit diluant, munie à son extrémité distale d'un embout et à son extrémité proximale d'une collerette, cette seringue étant apte par elle-même à recevoir un organe d'injection parentérale,
   - un support comprenant un corps de forme allongée et qui est rapporté sur la seringue, de préférence en l'entourant, ledit support étant ouvert à son extrémité distale et à son extrémité proximale, ledit support comprenant à son extrémité proximale des moyens de fixation sur la seringue, et, à son extrémité distale, au moins un moyen pour empêcher la connexion de l'organe d'injection parentérale sur l'embout de la seringue,
- ii) un flacon comprenant le principe actif sous forme sèche ou liquide et,
- iii) un système de transfert du diluant vers le principe actif, puis du produit médicamenteux reconstitué du flacon vers la seringue,
ledit système de transfert étant distinct dudit support.

Ainsi, grâce à l'ensemble selon l'invention, il est possible d'utiliser des seringues pré-remplies, par exemple des seringues pré-remplies standard en verre, pour une administration orale de produit sans risquer que le produit contenu dans la seringue soit administré par voie parentérale, et ce durant tout le temps de la manipulation de la seringue. De plus, le support entourant la seringue et étant solidaire de cette dernière tout le temps de la manipulation, et en particulier après la reconstitution du médicament et le retrait du système de transfert, il n'y a pas de risque que du verre par exemple, dans le cas d'une seringue en verre, ne vienne au contact de la bouche ou du corps du patient. Enfin, si la seringue est en verre, elle est protégée des chocs et donc des risques de brisure par le support.

Par exemple, dans le cas d'un produit médicamenteux nécessitant une reconstitution, le dispositif de l'ensemble selon l'invention rend impossible l'injection parentérale durant toutes les étapes de manipulation de la seringue, c'est-à-dire durant l'étape de connexion de la seringue au flacon comprenant le principe actif sous forme sèche ou liquide, puis pendant l'étape de reconstitution du produit médicamenteux et enfin pendant l'étape proprement dite d'administration du produit dans la bouche du patient, tout en autorisant la connexion du système de transfert sur la seringue pour la reconstitution du produit médicamenteux.

De même, dans le cas d'une seringue directement pré-remplie avec un médicament, l'injection parentérale de ce médicament est également rendue impossible grâce au dispositif de l'ensemble selon l'invention. Le dispositif de l'ensemble selon l'invention est ainsi totalement sécurisé. Grâce à ce dispositif, l'administration parentérale non souhaitée du produit contenu dans la seringue n'est pas possible.

Cet ensemble trouve une application en particulier pour l'administration orale de produits médicamenteux se présentant sous la forme de deux produits à mélanger : un premier produit, par exemple sous forme de poudre ou de lyophilisat, ou sous forme de liquide, est contenu dans un flacon et un deuxième produit, par exemple un diluant est contenu dans une seringue ainsi pré-remplie. Ainsi, tout le long de la préparation du produit médicamenteux et de son administration au patient, le risque d'injecter ledit produit par une voie autre qu'orale est absent.

Dans la présente demande, on entend par extrémité distale d'une pièce l'extrémité la plus éloignée de l'utilisateur du dispositif et par extrémité proximale, l'extrémité la plus proche de l'utilisateur du dispositif.

Dans la présente demande, on entend par « organe d'injection parentérale » tout organe permettant l'injection sous-cutanée ou intramusculaire ou intraveineuse d'un produit, tel qu'une aiguille, un cathéter, une ligne de perfusion ou tout autre système dont la connexion est telle que décrite par exemple dans la norme ISO 594-2.

La seringue de l'ensemble selon l'invention est généralement une seringue pré-remplie classique comprenant de façon générale un corps sensiblement tubulaire, un embout et une collerette.

La collerette de la seringue de l'ensemble selon l'invention peut être de forme variable. Elle peut être circulaire ou au contraire formée de parties radiales en regard les unes des autres. Elle peut n'être qu'un simple rebord, en particulier dans le cas où la seringue est une carpule.

La seringue peut être pré-remplie par un médicament sous forme liquide ou par un diluant de principe actif par-exemple.

Avantageusement, le support est fixé sur la seringue de façon définitive. Il n'est ainsi pas posssible à l'utilisateur de séparer le support de la seringue et d'utiliser la seringue pour une injection parentérale. Le dispositif est ainsi sécurisé.

De préférence, le support est fixé sur la seringue par collage, soudure, clipsage, encliquetage, sertissage ou encore déformation à chaud.

Dans une forme préférée de réalisation de l'invention, les moyens de fixation du support à la seringue se présentent sous la forme d'une rondelle comprenant une plateforme transversale et un rebord s'étendant dans le sens proximal et apte à se déformer et à se clipser sur la collerette de la seringue.

Dans une forme préférée de réalisation de l'invention, les moyens pour empêcher la connexion de l'organe d'injection parentérale sur l'embout de la seringue se présentent sous la forme d'une partie distale, située à l'extrémité distale du support, l'extrémité libre de ladite partie distale présentant une ouverture, de préférence axiale, dont le diamètre interne est strictement inférieur au diamètre externe de l'embase dudit organe d'injection parentérale.

Par diamètre interne, on entend, au sens de la présente demande, le diamètre du cercle inscrit dans la section de l'ouverture axiale de ladite partie distale. Cette section peut être de forme circulaire, ou sensiblement circulaire avec des décrochements. Dans une autre forme de réalisation de l'invention, cette section a une forme polygonale. De préférence encore, ce diamètre interne est inférieur ou égal à 7,70 mm. De préférence encore, la distance entre l'axe longitudinal de l'embout de la seringue et le point du périmètre de l'ouverture le plus proche de cet axe est inférieure ou égale à 3, 85 mm.

Par diamètre externe, on entend, au sens de la présente demande, le diamètre du cercle dans lequel est inscrite la plus grande section de l'embase de l'organe d'injection parentérale.

Dans une forme de réalisation de l'invention, la partie distale est une portion tronconique creuse convergeant dans le sens distal.

Dans une autre forme de réalisation de l'invention, la partie distale est un cylindre creux.

Dans une forme de réalisation de l'invention, le corps de la seringue est muni sur sa surface externe d'une pièce annulaire, par exemple en plastique, sur laquelle est fixé le support.

De préférence, la pièce annulaire est une bague à effet anti-retrait du piston. Cette bague empêche la sortie du piston de la seringue. De telles bagues sont décrites dans les documents EP 0 764 450, WO94/26334 et US 5, 803, 918.

Dans une forme préférée de réalisation de l'invention, le support est muni à son extrémité proximale d'une bague à effet anti-retrait du piston.

Dans une autre forme de réalisation de l'invention, le support est muni à son extrémité distale d'un capuchon lié au support par au moins un pont ruptible. Ce pont ruptible sert de témoin d'inviolabilité. En combinaison avec la bague anti-retrait du piston, le dispositif est ainsi totalement sécurisé au point de vue inviolabilité.

De préférence, le support du dispositif de l'ensemble selon l'invention est obtenu de moulage d'un matériau thermoplastique, tel que le polyéthylène, le polycarbonate ou le polypropylène.

Dans une forme de réalisation de l'invention, le corps du support comprend au moins une fenêtre longitudinale. Cette fenêtre permet l'observation du contenu de la seringue.

Dans une autre forme de réalisation de l'invention, le support est formé de deux parties longitudinales fixées l'une sur l'autre. De préférence, chaque partie longitudinale est obtenue de moulage d'un matériau thermoplastique, tel que le polyéthylène, le polycarbonate ou le polypropylène.

De préférence, les deux parties longitudinales sont fixées l'une sur l'autre par collage, soudure, clipsage, encliquetage, sertissage ou encore déformation à chaud, à l'aide d'une charnière ou par une combinaison de ces moyens.

Avantageusement, le support est muni d'une jupe transversale empêchant le dispositif de pénétrer trop avant dans la bouche du patient. Il n'y a ainsi pas de risque de blesser ce dernier.

Avantageusement, la seringue est en verre.

Un autre objet de l'invention porte sur l'utilisation d'un dispositif comprenant:
- une seringue pré-remplie avec un diluant, munie à son extrémité distale d'un embout et à son extrémité proximale d'une collerette, cette seringue étant apte par elle-même à recevoir un organe d'injection parentérale,
- un support comprenant un corps de forme allongée et qui est rapporté et fixé de façon définitive sur la seringue, de préférence en l'entourant, ledit support étant ouvert à son extrémité distale et à son extrémité proximale, ledit support comprenant à son extrémité proximale des moyens de fixation sur la seringue, ledit support comprenant en outre, à son extrémité distale, au moins un moyen pour empêcher la connexion de l'organe d'injection parentérale sur l'embout de la seringue,
pour rendre impossible, dans un ensemble pour l'administration orale d'un produit médicamenteux à reconstituer à partir d'un principe actif sous forme sèche ou liquide contenu dans un flacon et du diluant et comprenant un système de transfert, l'injection parentérale du diluant ou du produit médicamenteux reconstitué, durant toutes les étapes de manipulation de la seringue, tout en autorisant la connexion du système de transfert à la seringue.

Dans une forme de réalisation de l'invention, le système de transfert comprend à son extrémité distale une partie extrême apte à se connecter sur l'embout de la seringue une fois le support fixé sur ladite seringue.

Dans une forme de réalisation de l'invention, la partie extrême du système de transfert comporte des moyens de sécurisation du système de transfert sur le dispositif. La sécurité de l'ensemble selon l'invention est ainsi renforcée.

Dans une forme préférée de réalisation de l'invention, le moyen pour empêcher la connexion de l'organe d'injection parentérale sur l'embout de la seringue se présente sous la forme d'une partie distale située à l'extrémité distale du support, l'extrémité libre de ladite partie distale présentant une ouverture axiale comprenant au moins une encoche, de préférence au moins deux encoches, et de préférence encore deux encoches, les moyens de sécurisation du système de transfert se présentant sous la forme d'au moins un ergot, de préférence d'au moins deux ergots, et de préférence encore de deux ergots, apte(s) à coopérer avec la ou les encoche(s) de la partie distale du support. De préférence, l'ouverture axiale comprend deux encoches et les moyens de sécurisation se présentent sous la forme de deux ergots, les deux ergots étant décalés dans le sens axial ou de façon angulaire ou selon une combinaison de ces deux modes. Ainsi, un flacon non adapté, qui pourrait contenir une substance dangereuse ou non souhaitée, n'est pas utilisable dans l'ensemble selon l'invention qui est ainsi sécurisé.

Dans une forme de réalisation de l'invention, le système de transfert est serti sur le flacon de façon définitive. Dans ce cas, le système de transfert est inamovible et est muni d'une aiguille interne mobile activée lors de la connexion du système de transfert avec la seringue. Ainsi, il n'y a pas de risque que l'utilisateur se trompe de flacon. De tels systèmes sont décrits dans les documents US 5, 925, 029, US 6, 003, 566, US 6, 090, 093, US 6, 189, 580, US 6, 213, 994, US 6, 378, 576, US 6, 378, 714 et US 6, 382, 442.

Pour sa bonne compréhension, l'invention est décrite ci-dessous en référence au dessin schématique annexé, représentant, à titre d'exemple non limitatif, des formes de réalisation préférées du dispositif qu'elle concerne.
La figure 1 est une vue en perspective d'un dispositif d'un ensemble selon l'invention montrant une seringue pré-remplie installée dans un support,
La figure 2 est une vue en perspective de la seringue pré-remplie du dispositif selon la figure 1,
La figure 3 est une vue en perspective du support du dispositif selon la figure 1,
La figure 4 est une vue en perspective de la seringue du dispositif selon la figure 1, munie d'une bague à effet anti-retrait du piston,
La figure 5 est une vue en perspective de la bague à effet anti-retrait du piston de la seringue du dispositif selon la figure 4,
La figure 6 est une vue de dessus de la partie conique de l'extrémité distale du support selon la figure 3,
La figure 7 est une vue en perspective d'un support d'une variante d'un dispositif d'un ensemble selon l'invention,
La figure 8 est une vue en perspective d'une variante du dispositif comprenant un support selon la figure 7,
La figure 9 est une vue en perspective éclatée d'une autre variante d'un dispositif de l'ensemble selon l'invention,
La figure 10 est une vue en perspective du dispositif selon la figure 9 une fois le support fixé sur la seringue,
La figure 11 est une vue en perspective d'un organe d'injection parentérale,
La figure 12 est une vue en perspective illustrant l'impossibilité de connecter un organe d'injection parentérale à une seringue installée dans un support selon un dispositif de l'ensemble de l'invention,
Les figures 13 et 14 sont des vues en perspective d'une autre variante d'un dispositif de l'ensemble selon l'invention,
La figure 15 est une vue en perspective d'une autre variante dun dispositif de l'ensemble selon l'invention,
La figure 16 est une vue en coupe d'une partie du dispositif selon la figure 1,
La figure 17 est une vue de côté d'un système de transfert permettant le transfert d'un diluant et d'un principe actif entre le flacon et la seringue de l'ensemble selon l'invention,
La figure 18 est une vue en coupe d'un flacon contenant un principe actif à transférer dans la seringue du dispositif de l'ensemble selon l'invention avec l'aide du système de transfert de la figure 17,
La figure 19 est une vue en perspective du système de transfert de la figure 17 installé sur le flacon de la figure 18,
La figure 20 est une vue en perspective d'une variante de la figure 19 dans laquelle le système de transfert est serti sur le flacon,
Les figures 21 et 22 sont des vues en perspective montrant les moyens de sécurisation du système de transfert sur le dispositif de l'ensemble selon l'invention.

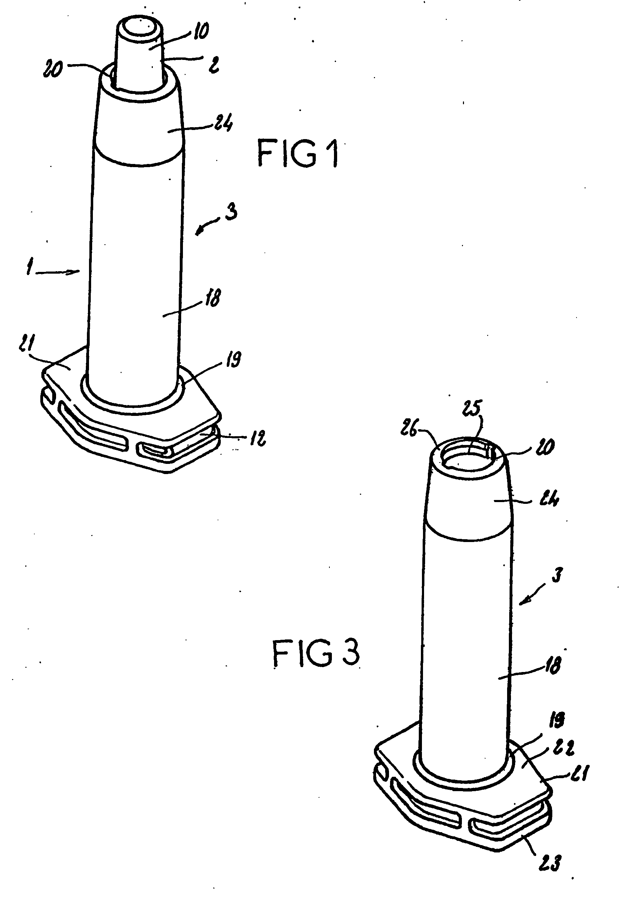

Les parties ou éléments du dispositif montrés sur les figures 1 à 22, qui se retrouvent de manière identique ou similaire sur les différentes variantes de réalisation, seront repérés par les mêmes références numériques et ne seront pas à nouveau décrits.

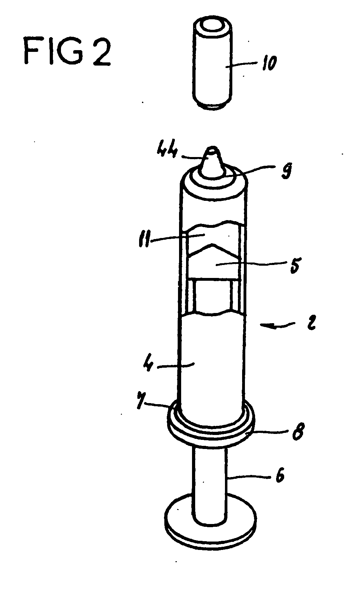

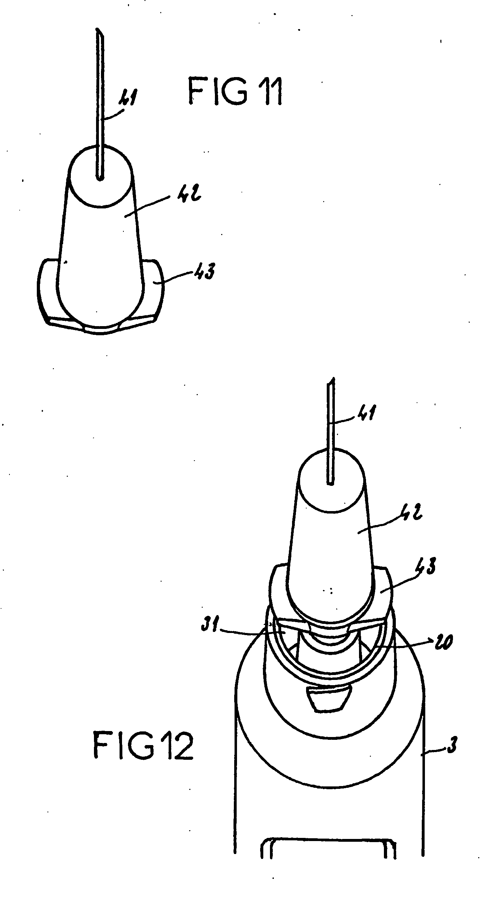

La figure 1 représente un dispositif 1 pour administration orale d'un produit médicamenteux comprenant une seringue 2 et un support 3 rapporté sur cette seringue 2 et l'entourant. Comme représenté sur la figure 2, la seringue 2 est une seringue pré-remplie par un liquide 11, comprenant à son extrémité distale 9, un embout 44 et à son extrémité proximale 7, une collerette 8. Cette seringue 2 est apte par elle-même à recevoir un organe 41 d'injection parentérale tel que par exemple représenté sur la figure 11. Sur les figures 1 et 2 est également représenté un capuchon 10 de bouchage et protection de l'embout 44. La seringue 2 comprend de manière classique un corps 4 de forme tubulaire et un piston constitué d'un joint de piston 5 et d'une tige de piston 6. Sur la figure 1, la seringue 2 est totalement recouverte par le support 3. Seul le capuchon 10 de l'embout 44 de la seringue 2 dépasse à l'extérieur du dispositif 1. La seringue 2, dans la configuration selon la figure 1, ne peut plus être connectée à un organe d'injection parentérale.

Sur la figure 3 est représenté le support 3 du dispositif 1 de la figure 1. Ce support 3 comprend un corps 18 de forme allongée apte à recevoir la seringue 2. Ce corps 18 est creux, de préférence cylindrique et sa surface interne a un diamètre adapté pour recevoir la seringue 2. Ce corps 18 est ouvert à son extrémité proximale 19, pour recevoir la seringue 2, et à son extrémité distale 20.

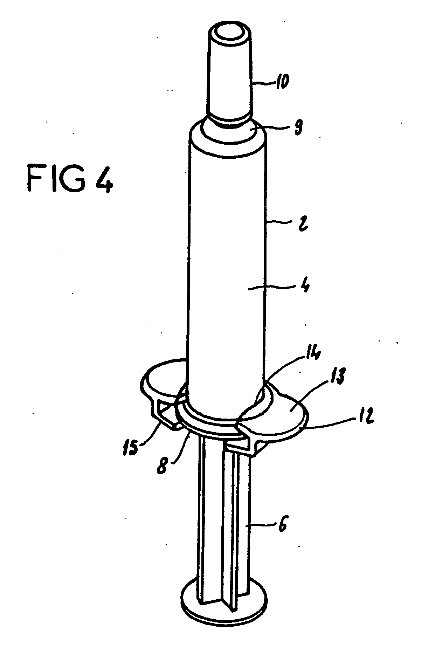

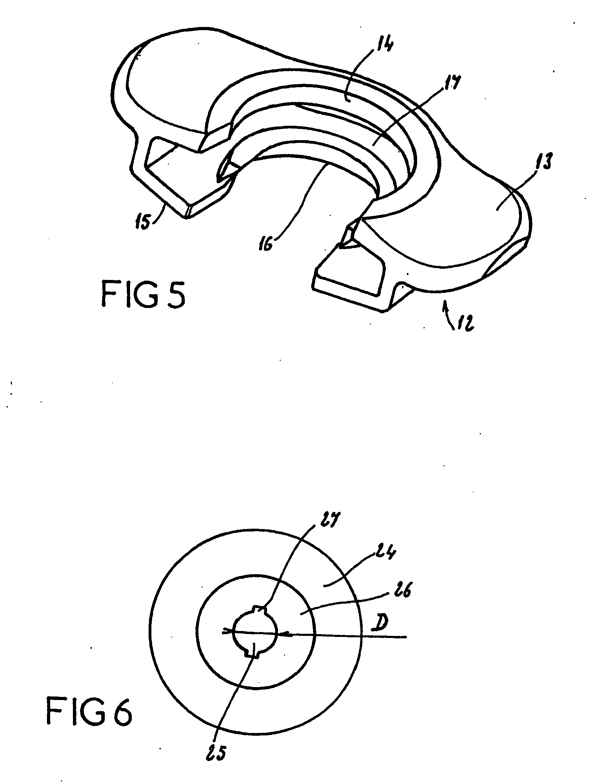

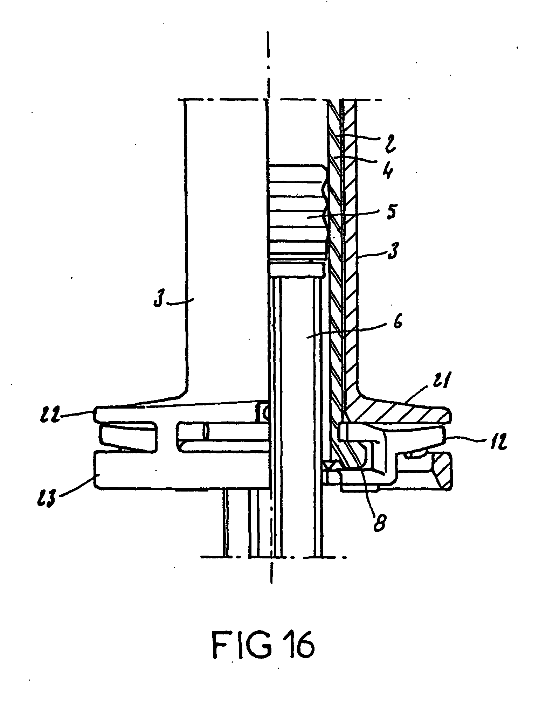

Le support 3 comprend à son extrémité proximale 19 des moyens de fixation 21, par exemple définitive, sur la seringue 2. Dans l'exemple représenté, ces moyens de fixation se présentent sous la forme d'une rondelle 21 comprenant une plateforme transversale 22 et un rebord 23 s'étendant dans le sens proximal et apte à se déformer et à se clipser, notamment définitivement, sur la collerette 8 de la seringue 2. Comme montré sur les figures 1 et 16, le rebord 23 peut aussi être apte à se clipser sur une bague 12 à effet anti-retrait du piston elle-même fixée sur la collerette 8 de la seringue 2 comme cela sera décrit ci-après en regard des figures 4 et 5.

Le support 3 comprend à son extrémité distale 20 au moins un moyen 24 pour empêcher la connexion d'un organe d'injection parentérale à l'embout 44 de la seringue 2. Sur la figure 3, ce moyen pour empêcher la connexion d'un organe d'injection parentérale à l'embout 44 de la seringue 2 se présente sous la forme d'une partie distale 24 située à l'extrémité distale 20 du support, l'extrémité libre 26 de ladite partie distale 24 présentant une ouverture axiale 25 dont le diamètre interne D, tel que représenté sur la figure 6, est strictement inférieur à celui du diamètre externe d'une embase d'un organe d'injection parentérale. Sur cette figure, cette partie distale 24 est une portion tronconique creuse convergeant dans le sens distal.

Sur les figures 1 et 16, le support 3 est fixé sur la seringue 2 par clipsage. En particulier, le support 3 est clipsé sur une bague 12 à effet anti-retrait du piston elle-même fixée sur la seringue 2 comme montré sur la figure 4.

Dans des variantes non représentées, le support 3 peut être fixé sur la seringue 2 par collage, soudure, encliquetage, sertissage ou déformation à chaud.

Sur la figure 4, est représentée une seringue 2 convenant au dispositif selon l'invention dont le corps 4 est muni sur sa surface externe d'une pièce annulaire 12. Dans l'exemple représenté, cette pièce annulaire 12 est une bague là effet anti-retrait du piston.

En se référant à la figure 5 cette bague 12 comporte de façon classique une plateforme distale 13 comprenant une ouverture semi-circulaire 14 correspondant aux dimensions du corps tubulaire 4 de la seringue 2. La bague 12 comprend également une plateforme proximale 15 comprenant une ouverture semi-circulaire 16 correspondant aux dimensions de la partie coulissante de la tige de piston 6 de la seringue 2. L'espace 17 délimité entre les deux plateformes 13 et 15 est destiné à recevoir la collerette 8 de la seringue 2.

Ainsi, lorsque la bague 12 est clipsée sur la collerette 8 de la seringue 2, comme représenté sur la figure 4, du fait de la dimension particulière de l'ouverture semi-circulaire 16 de la plateforme inférieure 15, il est impossible de retirer le piston de la seringue 2, ce piston étant alors bloqué par la bague 12.

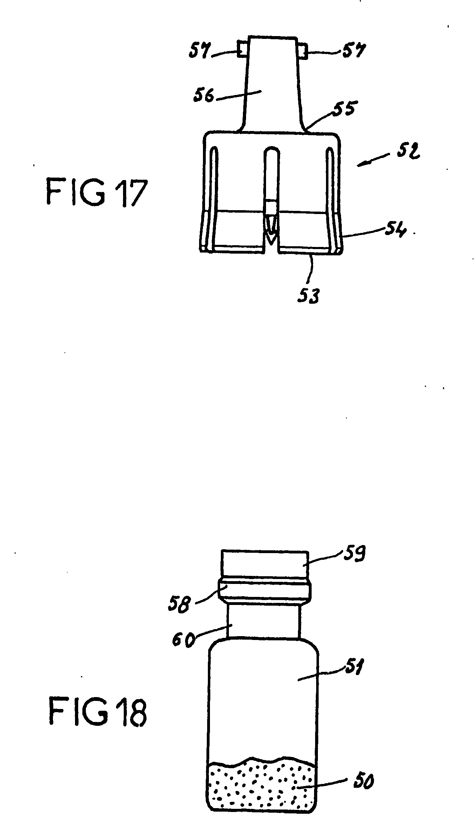

Sur la figure 6 est représentée l'ouverture axiale 25 de la portion tronconique 24 du support 3. Sur cette figure, le périmètre de l'ouverture 25 comprend deux encoches 27 destinées à permettre la connexion, par exemple par un système de baïonnette, de l'embout 44 de la seringue 2 à un système particulier de transfert relié au flacon comprenant un principe actif sous forme de poudre par exemple, système décrit aux figures 17 et 18. Dans une forme non représentée de réalisation de l'invention, l'ouverture 25 peut ne comprendre qu'une seule encoche 27 ou au contraire plus de deux encoches 27, aptes à coopérer avec un ou plusieurs ergots 57 (voir figure 17).

Le diamètre interne D est le diamètre du cercle inscrit dans la section de l'ouverture axiale 25.

Ce diamètre interne est strictement inférieur au diamètre externe d'une embase d'un organe d'injection parentérale.

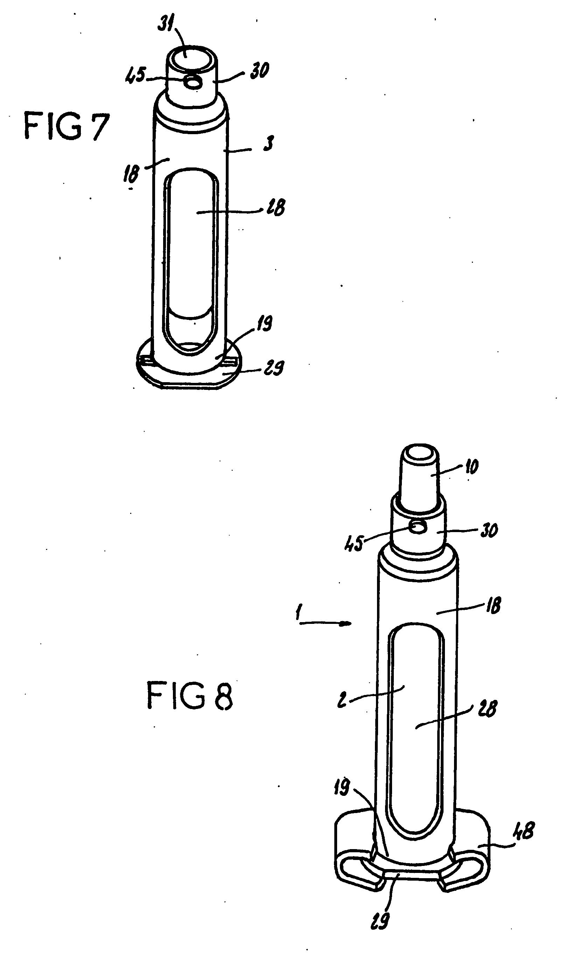

Sur la figure 7 est représentée une variante du support 3 d'un dispositif selon l'invention pour lequel le corps 18 du support 3 comprend au moins une fenêtre longitudinale afin de permettre l'observation du contenu de la seringue 2. Dans l'exemple représenté, le corps 18 comprend deux fenêtres longitudinales 28. Le corps 18 comprend à son extrémité proximale 19 un rebord annulaire 29 sur lequel une bague 48 (figure 8) à effet anti-retrait du piston et adaptée aux dimensions du corps 18 du support 3 peut se clipser. Le corps 18 du support 3 comprend à son extrémité distale 20 une partie distale 30, qui est un cylindre creux, et dont l'ouverture 31 présente un diamètre interne strictement inférieur à celui du diamètre externe de l'embase d'un organe d'injection parentérale.

Cette partie extrême cylindrique 30 comprend au moins un ergot extérieur 45 destiné à venir en coopération avec un système particulier de transfert (non représenté) lié à un flacon contenant un principe actif par exemple. Dans une variante de l'invention non représentée, l'ergot extérieur 45 est remplacé par une partie filetée destinée à venir en coopération avec le système de transfert.

Sur la figure 8 est représentée une variante du dispositif 1 selon l'invention dans lequel le support 3 est muni à son extrémité proximale 19 d'une bague 48 à effet anti-retrait du piston. Cette bague 48 à effet anti-retrait du piston est similaire à la bague 12 à effet anti-retrait du piston déjà décrite à la figure 5 mais, dans ce cas, les dimensions de l'ouverture semi-circulaire de la plateforme distale sont adaptées aux dimensions du corps 18 du support 3. La bague 48 à effet anti-retrait du piston est donc rajoutée une fois que le support 3 est installé sur la seringue 2. Cette bague 48 permet ainsi la fixation, notamment définitive, du support 3 sur la seringue 2.

Pour une meilleure compréhension, est représentée sur la figure 11 un organe d'injection parentérale sous la forme d'une aiguille 41 fixée sur son embase 42, par exemple de forme tronconique. Cette embase 42 comprend de façon classique une collerette 43. Le diamètre externe de l'embase 42 est donc le diamètre dans lequel est inscrite la section la plus grande de la collerette 43.

Comme représenté à la figure 12, le support 3 étant, notamment définitivement, fixé sur la seringue 2 et son extrémité distale 20 présentant une ouverture axiale 31 dont le diamètre interne est strictement inférieur au diamètre externe de l'embase 42 de l'organe d'injection parentérale 41, c'est-à-dire strictement inférieur au diamètre externe de la collerette 43, il est impossible de connecter l'organe d'injection parentérale, ici l'aiguille 41, sur la seringue 2.

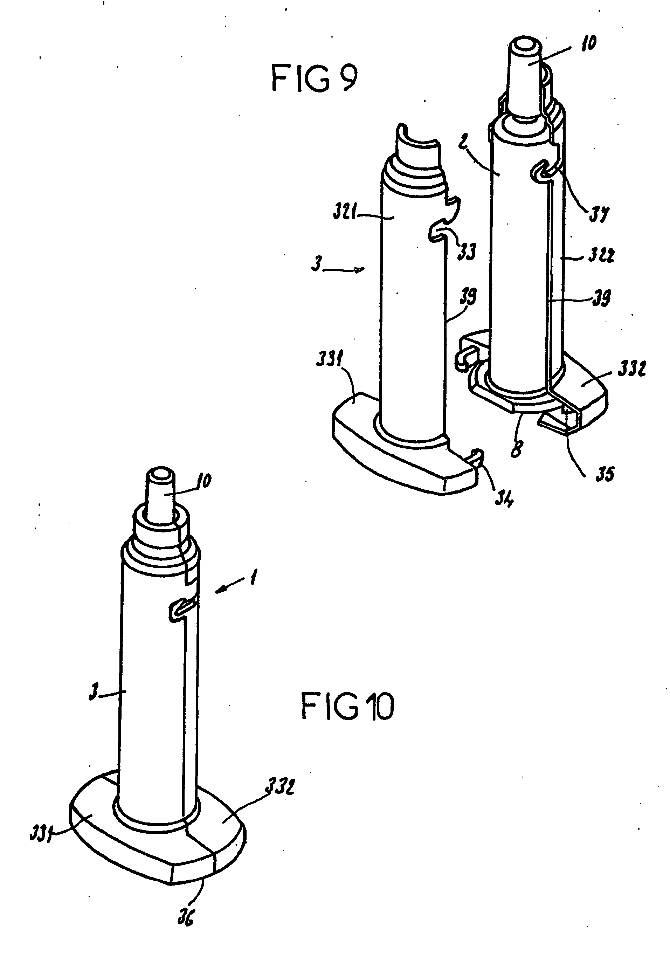

Sur les figures 9 et 10 est représentée une autre variante du dispositif 1 de l'ensemble selon l'invention dans laquelle le support 3 est formé de deux parties longitudinales 321, 322 fixées l'une à l'autre. Sur ces figures, ces deux parties longitudinales se présentent sous là forme de deux demi-coques 321 et 322 de forme semi-tubulaire aptes à être clipsées l'une sur l'autre. Chaque demi-coque 321, 322 du support 3 comprend à son extrémité proximale une demi-bague 331, 332 apte à être clipsée sur la demi-bague 331, 332 de l'autre demi-coque 321, 322. Des moyens de clipsage, tels qu'une saillie 34 et un évidement 35 sont prévus sur chaque demi-bague 331, 332 en regard d'un évidement 35 et d'une saillie 34 correspondants sur l'autre demi-bague 331, 332 afin de clipser les deux demi-bagues 331, 332 l'une sur l'autre. La bague 36 ainsi formée emprisonne la collerette 8 de la seringue 2 comme montré sur la figure 10. Cette bague 36 peut avoir un effet anti-retrait du piston.

Des moyens de clipsage, tels qu'une saillie 37 et un évidement 38 sont prévus sur les bords 39 respectifs de chaque demi-coque 321, 322 en regard d'un évidement 38 et d'une saillie 37 correspondants sur l'autre demi-coque 321, 322 afin de clipser les deux demi-coques 321, 322 l'une sur l'autre. Sur la figure 10, les deux demi-coques 321, 322 sont fixées l'une à l'autre par clipsage. Le support 3 ainsi formé emprisonne la seringue 2.

Dans des variantes non représentées, les deux demi coques 321, 322 peuvent être fixées l'une à l'autre par collage, soudure, encliquetage, sertissage, déformation à chaud, à l'aide d'une charnière ou par une combinaison de ces moyens.

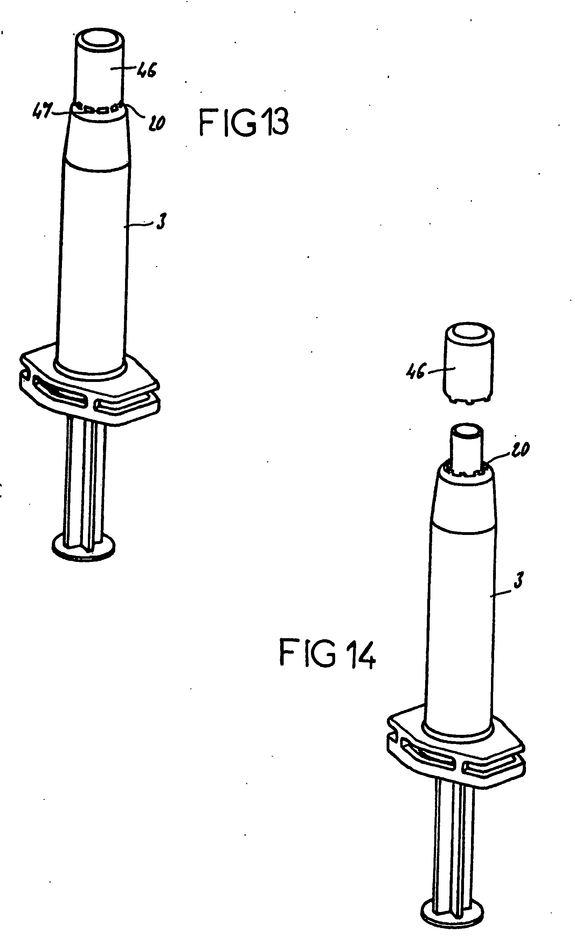

Sur les figures 13 et 14 est représentée une variante du dispositif de l'ensemble selon l'invention pour lequel le support 3 est muni à son extrémité distale 20 d'un capuchon 46 lié au support 3 par des ponts ruptibles 47. Ces ponts ruptibles servent de témoin d'inviolabilité.

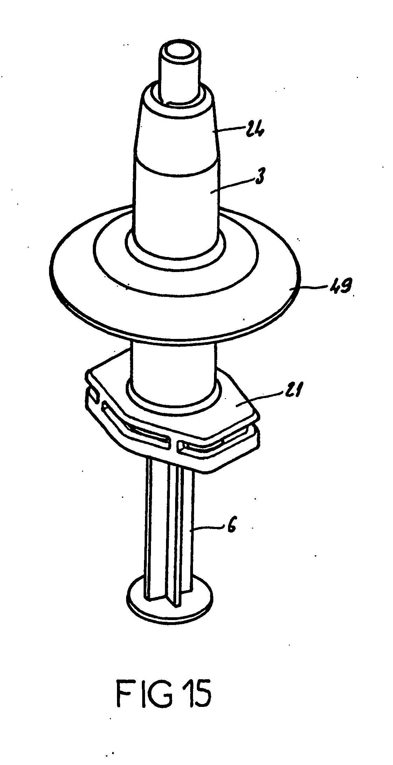

Sur la figure 15 est représentée une variante du dispositif de l'ensemble selon l'invention dans lequel le support 3 est muni d'une jupe transversale 49 empêchant le dispositif 1 de pénétrer trop avant dans la bouche du patient. Il n'y a ainsi aucun risque de blesser le patient.

Les figures 1, 2, 3, 17, 18 et 19 illustrent en combinaison un ensemble selon l'invention pour l'administration orale d'un produit médicamenteux à reconstituer à partir d'un principe actif 50 sous forme sèche ou liquide et d'un diluant 11. Cet ensemble comprend le dispositif 1 des figures 1 à 3 dans lequel la seringue 2 est pré-remplie avec le diluant 11, un flacon 51 comprenant le principe actif 50, sous forme sèche dans l'exemple représenté, et un système de transfert 52 du diluant 11 vers le principe actif 50, puis du produit médicamenteux reconstitué du flacon 51 vers la seringue 2. Le principe actif 50 peut être sous forme de poudre ou de lyophilisat. Le flacon 51 comprend un goulot 60 comprenant un simple rebord 58. Le flacon 51 est obturé par un bouchon 59 généralement maintenu en place par une bague sertie en aluminium (non représentée), munie d'un opercule que l'on peut ôter ou percer au moment de l'utilisation du flacon 51 avec le système de transfert 52. Le système de transfert 52 est distinct du support 3. Ce système de transfert 52 comprend à son extrémité distale 53 une base 54 creuse de forme tronconique s'évasant dans le sens distal. Cette base 54 comprend une nervure sur sa face intérieure destinée à se clipser sous le rebord 58 du goulot 60 du flacon 51.

Le système de transfert 52 comprend à son extrémité proximale 55 une partie extrême 56, en forme de portion tronconique dans l'exemple représenté, apte à se connecter sur l'embout 44 de la seringue 2 une fois le support 3 fixé sur ladite seringue 2. La partie extrême 56 du système de transfert 52 comporte des moyens 57 de sécurisation du système de transfert 52 sur le dispositif 1. Sur la figure 17, ces moyens 57 de sécurisatlon se présentent sous la forme de deux ergots 57 aptes à coopérer avec les encoches 27 de la partie distale 24 du support 3 comme représenté sur la figure 6.

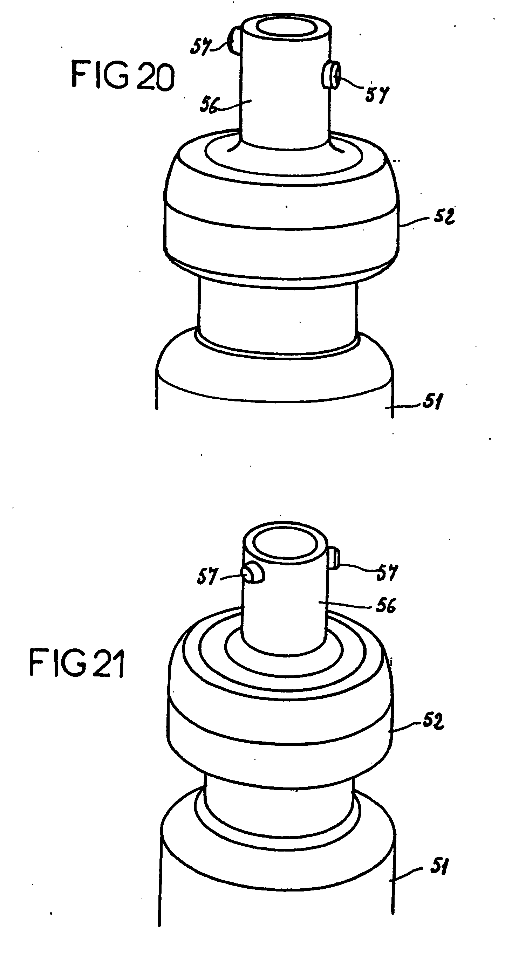

Comme représenté sur les figures 20 et 21, ces deux ergots 57 peuvent être décalés dans le sens axial (figure 20) ou de façon angulaire (figure 21) ou encore selon une combinaison de ces deux modes.

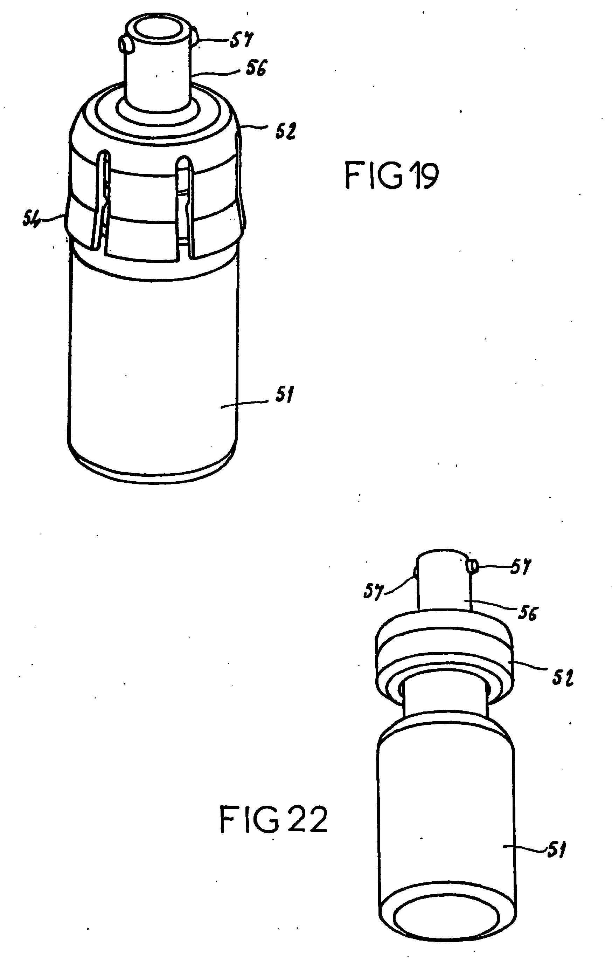

Ainsi, pour reconstituer un produit médicamenteux à partir de l'ensemble selon l'invention, l'utilisateur fixe le goulot 60 du flacon 51 sur la base 54 du système de transfert 52 en clipsant le rebord 58 du goulot 60 sur la nervure située sur la face intérieure de la base 54 du système de transfert 52. Il connecte ensuite le système de transfert 52, sur lequel est fixé le flacon 51 comme représenté sur la figure 19, sur l'embout 44 de la seringue 2 installée dans le support 3, grâce à la partie extrême 56 dudit système de transfert 52. Le système de transfert 52 est alors sécurisé sur le dispositif 1 grâce à la coopération des ergots 57 de la partie extrême 56 du système et des encoches 27 de l'ouverture de la partie distale 24 du support 3. Le diluant 11 de la seringue 2 est alors transféré dans le flacon 51 où il se mélange avec le principe actif 50 sous forme sèche (ou sous forme liquide), reconstituant le produit médicamenteux qui est alors ré-aspiré du flacon 51 dans la seringue 2. On déconnecte alors le système de transfert 52 du dispositif 1. Le système de transfert 52 étant distinct du support 3, ce dernier reste fermement fixé sur la seringue 2. Grâce à la partie distale 24 du support 3, la connexion de l'embout de la seringue 2 avec un organe d'injection parentérale est impossible. De plus, la seringue 2 est parfaitement recouverte par le support 3 qui l'entoure. Elle ne peut ainsi entrer en contact avec la bouche ou le corps du patient. Elle est également protégée par le support 3 contre les risques de brisure qui pourraient survenir suite à des chocs. Le produit médicamenteux peut ainsi être administré au patient par voie orale sans risque, même si la seringue 2 est en verre

Sur la figure 22, est représenté un système de transfert 52 serti de façon définitive sur le flacon 51. Il n'y a ainsi pas de risque pour l'utilisateur de connecter le système de transfert 52 à un flacon comportant un produit autre que le principe actif 50 que l'on souhaite mélanger au diluant 11 présent dans la seringue.

L'ensemble selon l'invention présente l'avantage de permettre l'utilisation de seringues pré-remplies, par exemple des seringues pré-remplies standard en verre, pour une administration orale de produit sans risque de blessure par mise en contact du corps en verre de la seringue avec une partie du corps du patient, et sans risquer que le produit contenu dans la seringue soit administré par voie parentérale, et ce durant tout le temps de la manipulation de la seringue.

La présente invention n'est pas limitée aux formes d'exécution décrites dans la présente demande à titre d'exemples.

## Revendications

1. Ensemble pour l'administration orale d'un produit médicamenteux à reconstituer à partir d'un principe actif (50) sous forme sèche ou liquide et d'un diluant (11), **caractérisé en ce qu'il** comprend :
- i) un dispositif (1) comprenant :
- une seringue (2) pré-remplie avec ledit diluant (11), munie à son extrémité distale (9) d'un embout et à son extrémité proximale (7) d'une collerette (8), cette seringue (2) étant apte par elle-même à recevoir un organe (41) d'injection parentérale,
- un support (3) comprenant un corps (18) de forme allongée et qui est rapporté sur la seringue (2), de préférence en l'entourant, ledit support (3) étant ouvert à son extrémité distale (20) et à son extrémité proximale (19), ledit support comprenant à son extrémité proximale (19) des moyens de fixation (21 ; 331, 332, 36 ; 48) sur la seringue (2), et, à son extrémité distale (20), au moins un moyen (24 ; 30) pour empêcher la connexion de l'organe d'injection parentérale (41) sur l'embout (44) de la seringue (2),
- ii) un flacon (51) comprenant le principe actif (50) sous forme sèche ou liquide, et
- iii) un système de transfert (52) du diluant (11) vers le principe actif (50), puis du produit médicamenteux reconstitué du flacon (51) vers la seringue (2),
ledit système de transfert (52) étant distinct dudit support (3).

2. Ensemble selon la revendication 1, **caractérisé en ce que** le support (3) est fixé sur la seringue (2) de façon définitive.

3. Ensemble selon la revendication 1 ou 2, **caractérisé en ce que** le support (3) est fixé à la seringue (2) par collage, soudure, clipsage, encliquetage, sertissage ou encore déformation à chaud.

4. Ensemble selon l'une quelconque des revendications précédentes, **caractérisé en ce que** les moyens de fixation du support à la seringue se présentent sous la forme d'une rondelle (21) comprenant une plateforme transversale (22) et un rebord (23) s'étendant dans le sens proximal et apte à se déformer et à se clipser sur la collerette (8) de la seringue (2).

5. Ensemble selon l'une quelconque des revendications précédentes, **caractérisé en ce que** les moyens pour empêcher la connexion de l'organe d'injection parentérale sur l'embout de la seringue se présentent sous la forme d'une partie distale (24 ; 30), située à l'extrémité distale (20) du support (3), l'extrémité libre (26) de ladite partie distale (24 ; 30) présentant une ouverture (25 ; 31), de préférence axiale, dont le diamètre interne est strictement inférieur au diamètre externe de l'embase (42) dudit organe d'injection parentérale (41).

6. Ensemble selon la revendication 5, **caractérisé en ce que** ledit diamètre interne est inférieur ou égal à 7,70 mm.

7. Ensemble selon la revendication 5 ou 6, **caractérisé en ce que** la distance entre l'axe longitudinal de l'embout (44) de la seringue et le point du périmètre de l'ouverture (25 ; 31) le plus proche de cet axe est inférieure ou égale à 3, 85 mm.

8. Ensemble selon l'une quelconque des revendications 5 à 7, **caractérisé en ce que** la partie distale est une portion tronconique creuse (24) convergeant dans le sens distal.

9. Ensemble selon l'une quelconque des revendications 5 à 7, **caractérisé en ce que** la partie distale est un cylindre creux (30).

10. Ensemble selon l'une quelconque des revendications précédentes, **caractérisé en ce que** le corps (4) de la seringue (2) est muni sur sa surface externe d'une pièce annulaire (12) sur laquelle est fixé le support (3).

11. Ensemble selon la revendication 10, **caractérisé en ce que** la pièce annulaire est une bague (12) à effet anti-retrait du piston.

12. Ensemble selon l'une quelconque des revendications précédentes, **caractérisé en ce que** le support (3) est formé de deux parties longitudinales (321, 322) fixées l'une à l'autre.

13. Ensemble selon la revendication 12, **caractérisé en ce que** les deux parties longitudinales (321, 322) sont fixées l'une sur l'autre par collage, soudure, clipsage, encliquetage, sertissage ou encore déformation à chaud, à l'aide d'une charnière ou par une combinaison de ces moyens.

14. Ensemble selon l'une quelconque des revendications précédentes, **caractérisé en ce que** le support (3) est muni à son extrémité distale (20) d'un capuchon (46) lié au support (3) par au moins un pont ruptible (47).

15. Ensemble selon l'une quelconque des revendications précédentes, **caractérisé en ce que** le support (3) est muni d'une jupe transversale (49) empêchant le dispositif de pénétrer trop avant dans la bouche du patient.

16. Ensemble selon l'une quelconque des revendications précédente, **caractérisé en ce que** le corps (18) du support (3) comprend au moins une fenêtre longitudinale (28).

17. Ensemble selon l'une quelconque des revendications précédentes, **caractérisé en ce que** le support (3) est muni à son extrémité proximale (19) d'une bague (48) à effet anti-retrait du piston.

18. Ensemble selon l'une quelconque des revendications précédentes, **caractérisé en ce que** la seringue (2) est en verre.

19. Ensemble selon l'une quelconque des revendications précédentes, **caractérisé en ce que** le système de transfert (52) comprend à son extrémité proximale (55) une partie extrême (56) apte à se connecter sur l'embout (44) de la seringue (2) une fois le support (3) fixé sur ladite seringue (2).

20. Ensemble selon l'une quelconque des revendications précédentes, **caractérisé en ce que** la partie extrême (56) du système de transfert (52) comporte des moyens (57) de sécurisation du système de transfert (52) sur le dispositif (1).

21. Ensemble selon la revendication précédente, **caractérisé en ce que** le moyen (24 ;30) pour empêcher la connexion de l'organe d'injection parentérale (41) sur l'embout (44) de la seringue (2) se présente sous la forme d'une partie distale située à l'extrémité distale (20) du support, l'extrémité libre (26) de ladite partie distale (24) présentant une ouverture axiale (25) comprenant au moins une encoche (27), de préférence au moins deux encoches (27), de préférence encore deux encoches (27), les moyens de sécurisation du système de transfert (52) se présentant sous la forme d'au moins un ergot (57), de préférence d'au moins deux ergots (57), de préférence encore de deux ergots (57) apte(s) à coopérer avec la ou les encoches (27) de la partie distale (24) du support (3).

22. Ensemble selon la revendication précédente, **caractérisé en ce que** l'ouverture axiale (25) comprend deux encoches (27) et les moyens de sécurisation se présentent sous la forme de deux ergots (57), les deux ergots (57) étant décalés dans le sens axial ou de façon angulaire ou selon une combinaison de ces deux modes.

23. Ensemble selon l'une quelconque des revendications précédentes, **caractérisé en ce que** le système de transfert (52) est serti sur le flacon (51) de façon définitive.

24. Utilisation d'un dispositif (1) comprenant :
- une seringue (2) pré-remplie avec un diluant (11), munie à son extrémité distale (9) d'un embout et à son extrémité proximale (7) d'une collerette (8), cette seringue (2) étant apte par elle-même à recevoir un organe (41) d'injection parentérale,
- un support (3) comprenant un corps (18) de forme allongée et qui est rapporté et fixé de façon définitive sur la seringue (2), de préférence en l'entourant, ledit support (3) étant ouvert à son extrémité distale (20) et à son extrémité proximale (19), ledit support comprenant à son extrémité proximale (19) des moyens de fixation (21 ; 331, 332, 36 ; 48) sur la seringue (2), ledit support comprenant en outre, à son extrémité distale (20), au moins un moyen (24 ; 30) pour empêcher la connexion de l'organe d'injection parentérale (41) sur l'embout (44) de la seringue (2),
pour rendre impossible, dans un Ensemble pour l'administration orale d'un produit médicamenteux à reconstituer à partir d'un principe actif (50) sous forme sèche ou liquide contenu dans un flacon (51) et du diluant (11) et comprenant un système de transfert (52), ledit système de transfert étant distinct dudit support, l'injection parentérale du diluant (11) ou du produit médicamenteux reconstitué, durant toutes les étapes de manipulation de la seringue (2), tout en autorisant la connexion du système de transfert (52) à la seringue (2).

## Claims

1. Assembly for oral administration of a medicinal product to be reconstituted from an active principle (50) in dry or liquid form and from a diluent (11), **characterized in that** it comprises :
- i) a device (1) comprising:
- a prefilled syringe (2) with said diluent (11), furnished at its distal end (9) with a tip and at its proximal end (7) with a collar (8), this syringe (2) being able by itself to receive a parenteral injection member (41),
- a support (3) comprising a body (18) of elongate form which is fastened to the syringe (2), preferably surrounding it, the said support (3) being open at its distal end (20) and at its proximal end (19), the said support comprising at its proximal end (19) means (21; 331; 332, 36; 48) for fixing to the syringe (2), and, at its distal end (20), at least one means (24; 30) for preventing the connecting of the parenteral injection member (41) to the tip (44) of the syringe (2),
- ii) a bottle (51) comprising the active principle (50) in dry or liquid form and
- iii) a system (52) for transferring the diluent (11) to the active principle (50), then the reconstituted medicinal product from the bottle (51) to the syringe (2),
said transfer system (52) being separate from the support (3).

2. Assembly according to Claim 1, **characterized in that** the support (3) is fixed definitively to the syringe (2).

3. Assembly according to Claim 1 or 2, **characterized in that** the support (3) is fixed to the syringe (2) by bonding, welding, clipping, snap-fastening, crimping or hot deformation.

4. Assembly according to any one of the preceding claims, **characterized in that** the means for fixing the support to the syringe take the form of a washer (21) comprising a transverse platform (22) and a rim (23) running in the proximal direction and able to deform and to clip to the collar (8) of the syringe (2).

5. Assembly according to any one of the preceding claims, **characterized in that** the means for preventing the connecting of the parenteral injection member to the tip of the syringe take the form of a distal part (24; 30), situated at the distal end (20) of the support (3), the free end (26) of the said distal part (24; 30) exhibiting an opening (25; 31), preferably axial, whose internal diameter is strictly less than the external diameter of the base (42) of the said parenteral injection member (41).

6. Assembly according to Claim 5, **characterized in that** the said internal diameter is less than or equal to 7.70 mm.

7. Assembly according to Claim 5 or 6, **characterized in that** the distance between the longitudinal axis of the tip (44) of the syringe and the point of the perimeter of the opening (25; 31) closest to this axis is less than or equal to 3.85 mm.

8. Assembly according to any one of Claims 5 to 7, **characterized in that** the distal part is a hollow tapered portion (24) converging in the distal direction.

9. Assembly according to any one of Claims 5 to 7, **characterized in that** the distal part is a hollow cylinder (30).

10. Assembly according to any one of the preceding claims, **characterized in that** the body (4) of the syringe (2) is furnished on its external surface with an annular piece (12) onto which the support (3) is fixed.

11. Assembly according to Claim 10, **characterized in that** the annular piece is a ring (12) with plunger anti-withdrawal effect.

12. Assembly according to any one of the preceding claims, **characterized in that** the support (3) is formed of two longitudinal parts (321, 322) fixed together.

13. Assembly according to Claim 12, **characterized in that** the two longitudinal parts (321, 322) are fixed together by bonding, welding, clipping, snap-fastening, crimping or hot deformation, with the aid of a hinge or through a combination of these means.

14. Assembly according to any one of the preceding claims, **characterized in that** the support (3) is furnished at its distal end (20) with a cap (46) linked to the support (3) by at least one breakable bridge (47).

15. Assembly according to any one of the preceding claims, **characterized in that** the support (3) is furnished with a transverse skirt (49) preventing the device from penetrating too far into the mouth of the patient.

16. Assembly according to any one of the preceding claims, **characterized in that** the body (18) of the support (3) comprises at least one longitudinal window (28).

17. Assembly according to any one of the preceding claims, **characterized in that** the support (3) is furnished at its proximal end (19) with a ring (48) with plunger anti-withdrawal effect.

18. Assembly according to any one of the preceding claims, **characterized in that** the syringe (2) is made of glass.

19. Assembly according to any one of the preceding claims, **characterized in that** the transfer system (52) comprises at its proximal end (55) an extreme part (56) able to connect to the tip (44) of the syringe (2) once the support (3) has been fixed to the said syringe (2).

20. Assembly according to any one of the preceding claims , **characterized in that** the extreme part (56) of the transfer system (52) comprises means (57) for making safe the transfer system (52) on the device (1).

21. Assembly according to the preceding claim, **characterized in that** the means (24; 30) for preventing the connecting of the parenteral injection member (41) to the tip (44) of the syringe (2) take the form of a distal part situated at the distal end (20) of the support, the free end (26) of the said distal part (24) exhibiting an axial opening (25) comprising at least one notch (27), preferably at least two notches (27), more preferably two notches (27), the securing means of the transfer system (52) taking the form of at least one lug (57), preferably at least two lugs (57), more preferably two lugs (57), able to cooperate with the notch(es) (27) of the distal part (24) of the support (3).

22. Assembly according to the preceding claim, **characterized in that** the axial opening (25) comprises two notches (27) and the securing means take the form of two lugs (57), these two lugs (57) being offset in the axial direction or in an angular manner or else according to a combination of these two modes.

23. Assembly according to any one of the preceding claims, **characterized in that** the transfer system (52) is definitively crimped to the bottle (51).

24. Use of a device (1) comprising:
- a prefilled syringe (2) with a diluent (11), furnished at its distal end (9) with a tip and at its proximal end (7) with a collar (8), this syringe (2) being able by itself to receive a parenteral injection member (41),
- a support (3) comprising a body (18) of elongate form which is fastened and fixed in a definitive way to the syringe (2), preferably surrounding it, the said support (3) being open at its distal end (20) and at its proximal end (19), the said support comprising at its proximal end (19) means (21; 331; 332, 36; 48) for fixing to the syringe (2), and, at its distal end (20), at least one means (24; 30) for preventing the connecting of the parenteral injection member (41) to the tip (44) of the syringe (2),
to render parenteral injection of the diluent (11) or of the reconstituted medicinal product impossible, in an assembly for oral administration of a medicinal product to be reconstituted from an active principle (50) in dry or liquid form contained in a bottle (51) and the diluent (11) and comprising a transfer system (52), said transfer system being separate from the support, during all the syringe (2) handling steps, while allowing the connection of the transfer system (52) to the syringe (2).

## Patentansprüche

1. Anordnung für die orale Verabreichung eines ausgehend von einem Wirkstoff (50) in trockener oder flüssiger Form und einem Verdünnungsmittel (11) aufzubauenden medikamentösen Erzeugnisses, **dadurch gekennzeichnet, dass** sie aufweist:
- i) eine Vorrichtung (1), mit:
- einer mit dem Verdünnungsmittel (11) vorgefüllten Spritze (2), die an ihrem distalen Ende (9) mit einer Tülle und an ihrem proximalen Ende (7) mit einem Flansch (8) versehen ist, wobei die Spritze (2) ihrerseits in der Lage ist, ein Organ (41) zur parenteralen Injektion aufzunehmen,
- einem Träger (3), der einen Körper (18) von länglicher Form aufweist und der an der Spritze (2) angebracht ist, wobei er sie vorzugsweise umgibt, wobei der Träger (3) an seinem distalen Ende (20) und an seinem proximalen Ende (19) offen ist, wobei der Träger an seinem proximalen Ende (19) Befestigungsmittel (21; 331, 332, 36; 48) zum Befestigen an der Spritze (2) und an seinem distalen Ende (20) zumindest ein Mittel (24; 30) aufweist, um die Verbindung des Organs (41) zur parenteralen Injektion an der Tülle (44) der Spritze (2) zu verhindern,
- ii) einem Fläschchen (51), das den Wirkstoff (50) in trockener oder flüssiger Form aufweist, und
- iii) einem Übertragungssystem (52) zum Übertragen des Verdünnungsmittels (11) zu dem Wirkstoff (50), dann des aufgebauten medikamentösen Erzeugnisses vom Fläschchen (51) zu der Spritze (2),
wobei das Übertragungssystem (52) von dem Träger (3) verschieden ist.

2. Anordnung nach Anspruch 1, **dadurch gekennzeichnet, dass** der Träger (3) an der Spritze (2) permanent befestigt ist.

3. Anordnung nach Anspruch 1 oder 2, **dadurch gekennzeichnet, dass** der Träger (3) an der Spritze (2) durch Kleben, Schweißen, Anklemmen, Verrasten, Einfassen oder auch durch Warmverformung befestigt ist.

4. Anordnung nach einem der vorhergehenden Ansprüche, **dadurch gekennzeichnet, dass** die Befestigungsmittel zum Befestigen des Trägers an der Spritze in Form einer Scheibe (21) ausgebildet sind, die eine quer verlaufende Plattform (21) und einen Rand (23) aufweist, der sich in proximaler Richtung erstreckt und in der Lage ist, sich zu verformen und sich an dem Flansch (8) der Spritze (2) anzuklemmen.

5. Anordnung nach einem der vorhergehenden Ansprüche, **dadurch gekennzeichnet, dass** die Mittel zum Verhindern der Verbindung des Organs zur parenteralen Injektion an der Tülle der Spritze in Form eines distalen Bereichs (24; 30) ausgebildet sind, der sich am distalen Ende (20) des Trägers (3) befindet, wobei das freie Ende (26) des distalen Bereichs (24; 30) eine Öffnung (25; 31) aufweist, die vorzugsweise axial ist, deren Innendurchmesser genau kleiner als der Außendurchmesser des Sockels (42) des Organs (41) zur parenteralen Injektion ist.

6. Anordnung nach Anspruch 5, **dadurch gekennzeichnet, dass** der Innendurchmesser kleiner oder gleich 7,70 mm ist.

7. Anordnung nach Anspruch 5 oder 6, **dadurch gekennzeichnet, dass** der Abstand zwischen der Längsachse der Tülle (44) der Spritze und dem Punkt des Umfangs der Öffnung (25; 31), der dieser Achse am nächsten liegt, kleiner oder gleich 3,85 mm ist.

8. Anordnung nach einem der Ansprüche 5 bis 7, **dadurch gekennzeichnet, dass** der distale Bereich ein hohler kegelstumpfförmiger Abschnitt (24) ist, der in distale Richtung konvergiert.

9. Anordnung nach einem der Ansprüche 5 bis 7, **dadurch gekennzeichnet, dass** der distale Bereich ein hohler Zylinder (30) ist.

10. Anordnung nach einem der vorhergehenden Ansprüche, **dadurch gekennzeichnet, dass** der Körper (4) der Spritze (2) an seiner Außenoberfläche mit einem ringförmigen Stück (12) versehen ist, an dem der Träger (3) befestigt ist.

11. Anordnung nach Anspruch 10, **dadurch gekennzeichnet, dass** das ringförmige Stück ein Ring (12) mit Anti-Abziehwirkung für den Kolben ist.

12. Anordnung nach einem der vorhergehenden Ansprüche, **dadurch gekennzeichnet, dass** der Träger (3) aus zwei längs verlaufenden Bereichen (321, 322) gebildet ist, die aneinander befestigt sind.

13. Anordnung nach Anspruch 12, **dadurch gekennzeichnet, dass** die zwei längs verlaufenden Bereiche (321, 322) aneinander durch Kleben, Schweißen, Anklemmen, Verrasten, Einfassen oder auch durch Warmverformung, mithilfe eines Scharniers oder durch eine Kombination dieser Mittel befestigt sind.

14. Anordnung nach einem der vorhergehenden Ansprüche, **dadurch gekennzeichnet, dass** der Träger (3) an seinem distalen Ende (20) mit einer Kappe (46) versehen ist, die mit dem Träger (3) durch zumindest eine brechbare Brücke (47) verbunden ist.

15. Anordnung nach einem der vorhergehenden Ansprüche, **dadurch gekennzeichnet, dass** der Träger (3) mit einer quer verlaufenden Schürze (49) versehen ist, die verhindert, dass die Vorrichtung zu weit nach vorn in den Mund des Patienten eindringt.

16. Anordnung nach einem der vorhergehenden Ansprüche, **dadurch gekennzeichnet, dass** der Körper (18) des Trägers (3) zumindest ein längs verlaufendes Fenster (28) aufweist.

17. Anordnung nach einem der vorhergehenden Ansprüche, **dadurch gekennzeichnet, dass** der Träger (3) an seinem proximalen Ende (19) mil einem Ring (48) mit Anti-Abziehwirkung für den Kolben versehen ist.

18. Anordnung nach einem der vorhergehenden Anspruche, **dadurch gekennzeichnet, dass** die Spritze (2) aus Glas ist.

19. Anordnung nach einem der vorhergehenden Ansprüche, **dadurch gekennzeichnet, dass** das Übertragungssystem (52) an seinem proximalen Ende (55) einen äußersten Bereich (56) aufweist, der in der Lage ist, sich mit der Tülle (44) der Spritze (2) zu verbinden, wenn der Träger (3) an der Spritze (2) befestigt ist.

20. Anordnung nach einem der vorhergehenden Ansprüche, **dadurch gekennzeichnet, dass** der äußerste Bereich (56) des Übertragungssystems (52) Sicherungsmittel (57) zum Sichern des Übertragungssystems (52) an der Vorrichtung (1) aufweist.

21. Anordnung nach dem vorhergehenden Anspruch, **dadurch gekennzeichnet, dass** das Mittel (24; 30) zum Verhindern der Verbindung des Organs (41) zur parenteralen Injektion an der Tülle (44) der Spritze (2) in Form eines distalen Bereichs ausgebildet ist, der sich am distalen Ende (20) des Trägers befindet, wobei das freie Ende (26) des distalen Bereichs (24) eine axiale Öffnung (25) aufweist, die zumindest eine Nut (27), vorzugsweise zumindest zwei Nuten (27), weiter vorzugsweise zwei Nuten (27) aufweist, wobei die Sicherungsmittel zum Sichern des Übertragungssystems (52) in Form zumindest eines Vorsprungs (57), vorzugsweise zumindest zweier Vorsprünge (57), weiter vorzugsweise zweier Vorsprünge (57) ausgebildet sind, der bzw. die in der Lage ist bzw. sind, mit der oder den Nuten (27) des distalen Bereichs (24) des Trägers (3) zusammenzuwirken.

22. Anordnung nach dem vorhergehenden Anspruch, **dadurch gekennzeichnet, dass** die axiale Öffnung (25) zwei Nuten (27) aufweist und die Sicherungsmittel in Form zweier Vorsprünge (57) ausgebildet sind, wobei die zwei Vorsprünge (57) in axialer Richtung oder winkelmäßig oder gemäß einer Kombination dieser beiden Arten versetzt sind.

23. Anordnung nach einem der vorhergehenden Ansprüche, **dadurch gekennzeichnet, dass** das Übertragungssystem (52) an dem Fläschchen (51) permanent eingefasst ist.

24. Verwendung einer Vorrichtung (1), mit:
- einer Spritze (2), die mit einem Verdünnungsmittel (11) vorgefüllt ist, und die an ihrem distalen Ende (9) mit einer Tülle und an ihrem proximalen Ende (7) mit einem Flansch (8) versehen ist, wobei die Spritze (2) ihrerseits in der Lage ist, ein Organ (41) zur parenteralen Injektion aufzunehmen,
- einem Träger (3), der einen Körper (18) von länglicher Form aufweist, und der an der Spritze (2) angebracht und permanent befestigt ist, indem er sie vorzugsweise umgibt, wobei der Träger (3) an seinem distalen Ende (20) und an seinem proximalen Ende (19) offen ist, wobei der Träger an seinem proximalen Ende (19) Befestigungsmittel (21; 331, 332, 36; 48) zum Befestigen an der Spritze (2) aufweist, wobei der Träger außerdem an seinem distalen Ende (20) zumindest ein Mittel (24; 30) aufweist, um die Verbindung des Organs (41) zur parenteralen Injektion an der Tülle (44) der Spritze (2) zu verhindern,
um in einer Anordnung zur oralen Verabreichung eines ausgehend von einem Wirkstoff (50) in trockener oder flüssiger Form, der in einem Fläschchen (51) enthalten ist, und einem Verdünnungsmittel (11) aufzubauenden medikamentösen Erzeugnisses, und die ein Übertragungssystem (52) aufweist, wobei das Übertragungssystem von dem Träger verschieden ist, die parenterale Injektion des Verdünnungsmittels (11) oder des aufgebauten medikamentösen Erzeugnisses während aller Schritte der Handhabung der Spritze (2) unmöglich zu machen, und dabei die Verbindung des Übertragungssystems (52) an der Spritze (2) zu erlauben.
